# EUROPEAN PATENT APPLICATION

(11) **EP 3 932 942 A1**
(43) Date of publication of application: **05.01.2022**
(21) Application number: 20767092.8
(22) Date of filing: 14.01.2020
(51) Int. Cl.: C07K 14/435, C07K 16/18, G01N 33/53, G01N 33/543

(54) **METHOD AND KIT FOR MEASURING APP669-711**

(30) Priority: 01.03.2019 JP 2019037265
(71) Applicant: SHIMADZU CORPORATION, Kyoto-shi, Kyoto 604-8511 (JP)
(72) Inventor: KANEKO, Naoki, Kyoto-shi, Kyoto 604-8511 (JP)
(74) Representative: Müller-Boré & Partner Patentanwälte PartG mbB
(86) International application number: PCT/JP2020/000787
(87) International publication number: WO 2020/179224

(57) **Abstract**

A method of measuring APP669-711, comprising: an immobilization step involving immobilizing, to a support, a first antibody capable of immunospecifically binding to an N terminus of APP669-711; a blocking step involving blocking the support to which the first antibody is immobilized, with a blocking agent; a first reaction step involving bringing APP669-711 in the sample into contact with the first antibody thus immobilized, and thereby allowing APP669-711 to bind to the first antibody; a sample removal step involving removing the sample that is not bound; a second reaction step involving bringing a second antibody capable of immunospecifically binding to a C terminus of APP669-711, into contact with APP669-711 bound to the first antibody thus immobilized, and thereby allowing the second antibody to bind to APP669-711; a second antibody removal step involving removing the second antibody that is not bound; and a detection step involving detecting the presence of the second antibody bound to APP669-711.

SEQUENCE LISTING

<110>  SHIMADZU CORPORATION

<120>  Measurement Method For APP669-711 And Measurement Kit

<130>  9191065WO01

<150>  JP 2019-037265
<151>  2019-03-01

<160>  5

<210>  1
<211>  40
<212>  PRT
<213>  Homo Sapiens

<400>  1

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  2
<211>  42
<212>  PRT
<213>  Homo Sapiens

<400>  2

<210>  3
<211>  43
<212>  PRT
<213>  Homo Sapiens

<400>  3

Val Lys Met Asp Ala Glu Phe Arg His Asp Ser Gly Tyr Glu Val His
1               5                   10                  15

His Gln Lys Leu Val Phe Phe Ala Glu Asp Val Gly Ser Asn Lys Gly
            20                  25                  30

Ala Ile Ile Gly Leu Met Val Gly Gly Val Val
        35                  40

<210>  4
<211>  10
<212>  PRT
<213>  Homo Sapiens

<400>  4

Glu Val Lys Met Asp Ala Glu Phe Arg His
1               5                   10

<210>  5
<211>  4
<212>  PRT
<213>  Homo Sapiens

<400>  5

Val Lys Met Cys
1

## Description

### TECHNICAL FIELD

The present invention relates to a method and a kit for measuring APP669-711.

### BACKGROUND ART

Alzheimer's disease (AD) is a primary cause of dementia, accounting for 50% to 60% of the entire dementia population. The number of dementia patients worldwide is estimated to increase from 24 million in 2001 to reach 81 million in 2040. It is believed that the onset of Alzheimer's disease is deeply involved with Aβ. Aβ is produced as a result of proteolysis of amyloid precursor protein (APP), which is a single-pass transmembrane protein made up of 770 amino acid residues, by β-secretase and γ-secretase. Aβ undergoes fibrosis and aggregation to form senile plaques, which trigger aggregation and deposition of tau protein within neuronal cells, leading to neuronal dysfunction and/or neuronal cell death. This is believed to cause a significant loss of cognitive ability. It has long been known that Aβ essentially consists of a 40mer (Aβ1-40) and a 42mer (Aβ1-42), and also it is found that Aβ migrates into cerebrospinal fluid (CSF). Its potential migration into blood is also suggested. Further, a recent report shows the presence of an Aβ-like peptide in CSF, having a length different from that of Aβ1-40 or Aβ1-42.

As for Aβ, the inventors of the present invention reported through a recent research that the ratio of APP669-711 (an Aβ-related peptide (an Aβ-like peptide)) to Aβ1-42 holds great promise as a blood biomarker (NPL 1, PTL 1 (International Patent Laying-Open No. WO2015/178398)). The Aβ and Aβ-related peptide are quantified by immunoprecipitation (IP) accompanied by subsequent mass spectrometry (MALDI-TOF MS).

Further, the inventors of the present invention disclosed in PTL 2 (International Patent Laying-Open No. WO2017/047529) another promising blood biomarker, which is a numerical value obtained by mathematically combining two or more ratios selected from the group consisting of three ratios regarding Aβ and Aβ-related peptide (Aβ-like peptide), that are Aβ1-39/Aβ1-42, Aβ1-40/Aβ1-42, and APP669-711/Aβ1-42. The Aβ and Aβ-related peptides are quantified by immunoprecipitation (IP) accompanied by subsequent mass spectrometry (MALDI-TOF MS).

As shown above, the inventors of the present invention employed mass spectrometry (MALDI-TOF MS) to find that the ratio of APP669-711 (an Aβ-related peptide) to Aβ1-42 holds great promise as a blood biomarker candidate. In addition, the inventors of the present invention demonstrated, by using multiple samples from Japan and Australia, that a composite biomarker obtained by combining APP669-711/Aβ1-42 and Aβ1-40/Aβ1-42 ratios enables accurate prediction of cerebral amyloid deposition, and they also reported that this composite biomarker is a reliable, versatile biomarker (NPL 2).

Mass spectrometry (MALDI-TOF MS) is a useful analytical technique for analyzing biomarkers such as Aβ-related peptides including APP669-711. Another potentially useful analytical technique is sandwich ELISA, which is a cost-effective assay method widely used for laboratory tests.

Currently, ELISA Kits capable of analyzing plasma Aβ1-40 and/or Aβ1-42 are commercially available from various manufacturers (such as Wako Pure Chemical Industries and IBL).

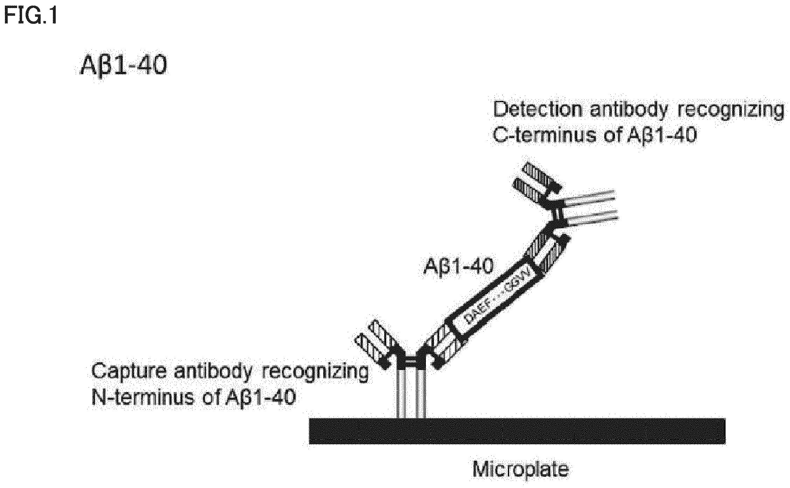

Fig. 1 is a schematic view of a sandwich ELISA method for Aβ1-40. Referring to Fig. 1, the N terminus of Aβ1-40 is captured by an antibody fixed on a microplate capable of specifically recognizing the N terminus of Aβ1-40, and the C terminus of Aβ1-40 is bound to a detection antibody capable of specifically recognizing the C terminus of Aβ1-40. Detecting this detection antibody enables measuring Aβ1-40.

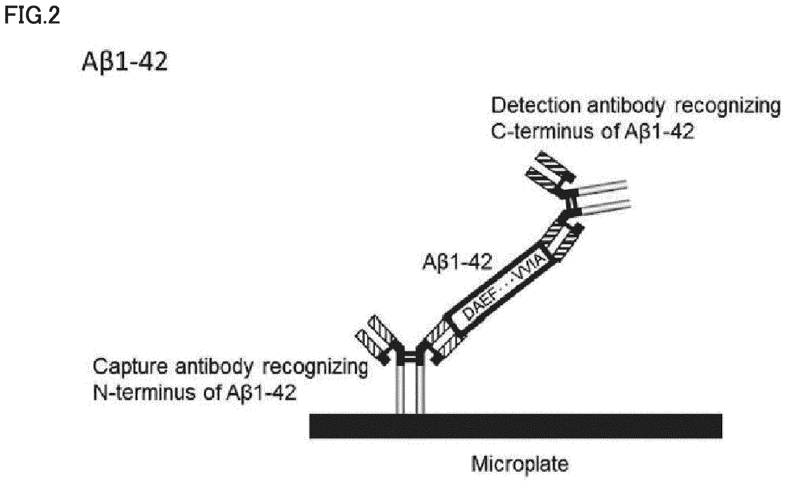

Fig. 2 is a schematic view of a sandwich ELISA method for Aβ1-42. Referring to Fig. 2, the N terminus of Aβ1-42 is captured by an antibody fixed on a microplate capable of specifically recognizing the N terminus of Aβ1-42, and the C terminus of Aβ1-42 is bound to a detection antibody capable of specifically recognizing the C terminus of Aβ1-42. Detecting this detection antibody enables measuring Aβ1-42.

PTL 3 (Japanese Patent Laying-Open No. 2005-170951) discloses monoclonal antibodies BAN-52a and BAN-50a capable of recognizing the N terminus of amyloid β (Aβ1-16) and monoclonal antibodies BA-27a, BS-85, and BC-05a capable of specifically recognizing the C terminus of amyloid β, and also discloses a sandwich EIA specific to Aβ1-40 and Aβ1-42.

PTL 4 (Japanese Patent Laying-Open No. 2014-208678) discloses an antibody capable of specifically recognizing Aβ11-x, and also discloses sandwich ELISA for Aβ11-40 and Western blotting for Aβ11-x.

However, there is no known means for analyzing APP669-x by an immunoassay method.

### CITATION LIST

### PATENT LITERATURE

- PTL 1:: International Patent Laying-Open No. WO2015/178398
- PTL 2:: International Patent Laying-Open No. WO2017/047529
- PTL 3:: Japanese Patent Laying-Open No. 2005-170951
- PTL 4:: Japanese Patent Laying-Open No. 2014-208678

### NONPATENT LITERATURE

NPL 1: Kaneko N, Nakamura A, Washimi Y, Kato T, Sakurai T, Arahata Y, Bundo M, Takeda A, Niida S, Ito K, Toba K, Tanaka K, Yanagisawa K.: Novel plasma biomarker surrogating cerebral amyloid deposition. Proc Jpn Acad Ser B Phys Biol Sci. 2014; 90 (9): 353-364.

NPL 2: Nakamura A, Kaneko N, Villemagne VL, Kato T, Doecke J, Dore V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K.: High performance plasma amyloid-β biomarkers for Alzheimer's disease. Nature. 2018; 554 (7691): 249-254.

### SUMMARY OF INVENTION

### TECHNICAL PROBLEM

To develop a sandwich ELISA specific to a peptide, it is required to obtain antibodies that specifically recognize the N terminus and the C terminus, respectively. The C terminus of APP669-711 is the same as the C terminus of Aβ1-40 (namely, APP672-711), and there exists an antibody capable of specifically recognizing the C terminus of APP669-711. However, there exists no antibody capable of specifically recognizing the N terminus of APP669-711.

Since there exists no antibody capable of recognizing the N terminus of APP669-711, there exists no analytical means based on a sandwich ELISA method capable of specifically quantifying APP669-711.

To address this problem, an object of the present invention is to prepare an antibody capable of recognizing the N terminus of peptide APP669-711 related with amyloid β (Aβ) and an antibody capable of recognizing the C terminus of the same and provide an assay method for specifically quantifying APP669-711 [in particular, a sandwich ELISA method (Enzyme-Linked Immuno Sorbent Assay)]. Another object of the present invention is to provide a kit for measuring APP669-711 [in particular, a sandwich ELISA kit].

### SOLUTION TO PROBLEM

As a result of intensive research, the inventors of the present invention have successfully prepared an antibody capable of recognizing the N terminus of APP669-711 by immunizing a mouse with KLH (keyhole limpet hemocyanin) -bound synthetic peptide VKMC and then performing cell fusion and screening to obtain a hybridoma that produces an antibody capable of recognizing the N terminus of APP669-711. Using this antibody, they have developed a sandwich ELISA capable of specifically quantifying APP669-711. In this way, an ELISA kit for APP669-711 has been developed.

The present invention is basically based on a novel antibody, which is an APP669-711-N-terminus-recognizing monoclonal antibody capable of specifically recognizing the N terminus of APP669-711 peptide.

A first aspect of the present invention relates to a method of measuring APP669-711 in a sample, comprising;
an immobilization step involving immobilizing, to a support, a first antibody capable of immunospecifically binding to an N terminus of APP669-711;
a blocking step involving blocking the support to which the first antibody is immobilized, with a blocking agent;
a first reaction step involving bringing APP669-711 in the sample into contact with the first antibody thus immobilized, and thereby allowing APP669-711 to bind to the first antibody;
a sample removal step involving removing the sample that is not bound;
a second reaction step involving bringing a second antibody capable of immunospecifically binding to a C terminus of APP669-711, into contact with APP669-711 bound to the first antibody thus immobilized, and thereby allowing the second antibody to bind to APP669-711;
a second antibody removal step involving removing the second antibody that is not bound; and
a detection step involving detecting the presence of the second antibody bound to APP669-711.

A second aspect of the present invention relates to a kit for measuring APP669-711, comprising:
an APP669-711-N-terminus-recognizing monoclonal antibody capable of specifically recognizing an N terminus of APP669-711 (a first antibody);
a monoclonal antibody or a polyclonal antibody capable of recognizing a C terminus of APP669-711 (a second antibody); and
a blocking agent.

### ADVANTAGEOUS EFFECTS OF INVENTION

The present invention provides a sandwich immunoassay method, particularly a sandwich ELISA method, for APP669-711, using an antibody capable of specifically recognizing the N terminus of APP669-711.

The present invention also provides a kit for measuring APP669-711, particularly a reagent kit for implementing a sandwich ELISA method. As an analytical technique for analyzing biomarkers such as Aβ-related peptides including APP669-711, mass spectrometry (MALDI-TOF MS) is useful but sandwich ELISA can be even more useful because it is a cost-effective assay method widely used for laboratory tests.

### BRIEF DESCRIPTION OF DRAWINGS

Fig. 1 is a schematic view of a sandwich ELISA method for Aβ1-40.
Fig. 2 is a schematic view of a sandwich ELISA method for Aβ1-42.

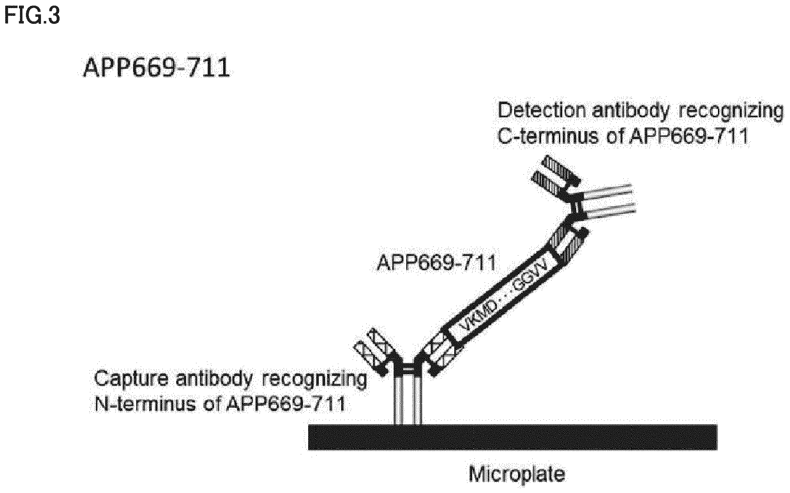

Fig. 3 is a schematic view of a sandwich ELISA method for APP669-711.

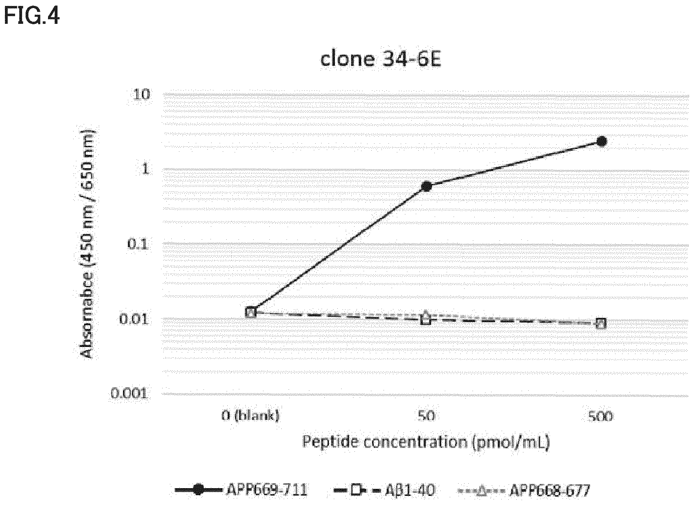

Fig. 4 is a graph showing results of direct ELISA using APP669-711-N-terminus-specific antibody clone 34-6E in Experiment Example 1-2. The horizontal axis represents the peptide concentration (pmol/mL), and the vertical axis represents the absorbance at 450 nm (primary wavelength)/650 nm (secondary wavelength) measured with a microplate reader.

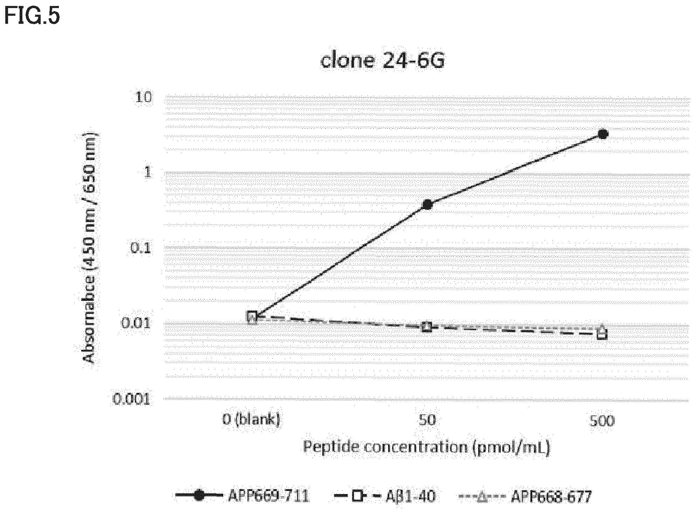

Fig. 5 is a graph showing results of direct ELISA using APP669-711-N-terminus-specific antibody clone 24-6G in Experiment Example 1-2. The horizontal axis represents the peptide concentration (pmol/mL), and the vertical axis represents the absorbance at 450 nm (primary wavelength)/650 nm (secondary wavelength) measured with a microplate reader.

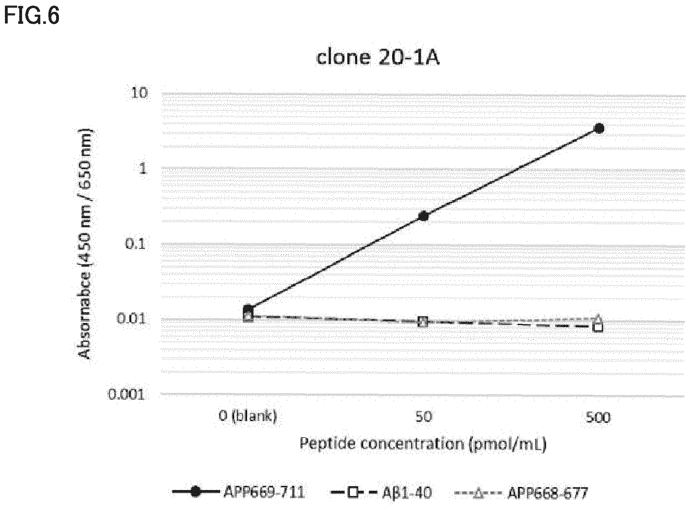

Fig. 6 is a graph showing results of direct ELISA using APP669-711-N-terminus-specific antibody clone 20-1A in Experiment Example 1-2. The horizontal axis represents the peptide concentration (pmol/mL), and the vertical axis represents the absorbance at 450 nm (primary wavelength)/650 nm (secondary wavelength) measured with a microplate reader.

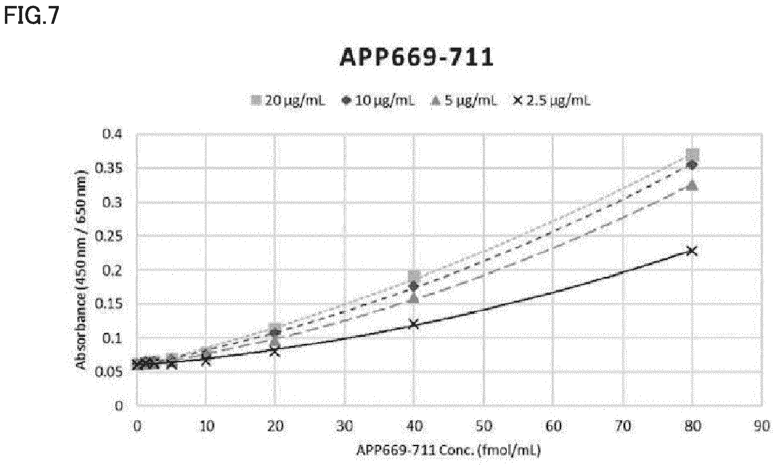

Fig. 7 is a graph showing APP669-711 ELISA reactivity where the concentration of a capture antibody solution during a step of fixing an APP669-711-N-terminus-recognizing antibody was changed in Example 1. The horizontal axis represents the concentration (fmol/mL) of APP669-711 standard liquid, and the vertical axis represents the absorbance at 450 nm (primary wavelength)/650 nm (secondary wavelength) measured with a microplate reader.

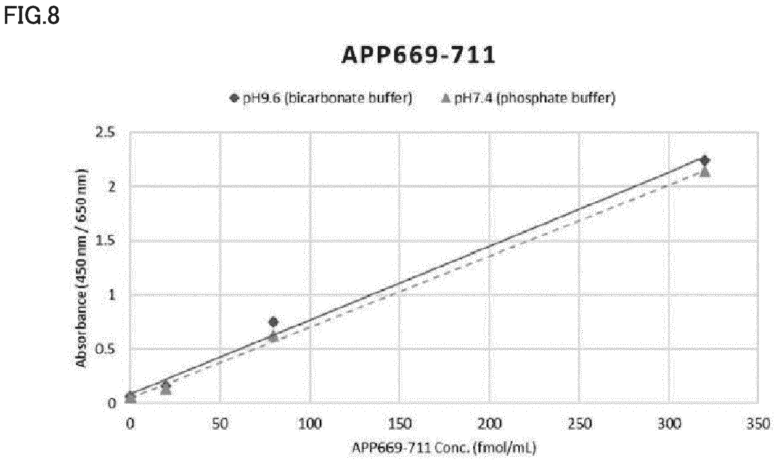

Fig. 8 is a graph showing APP669-711 ELISA reactivity when the pH of a capture antibody solution was 9.6 or 7.4 during a step of fixing an APP669-711-N-terminus-recognizing antibody in Example 2, where the horizontal axis represents the concentration (fmol/mL) of APP669-711 standard liquid, and the vertical axis represents the absorbance at 450 nm/650 nm.

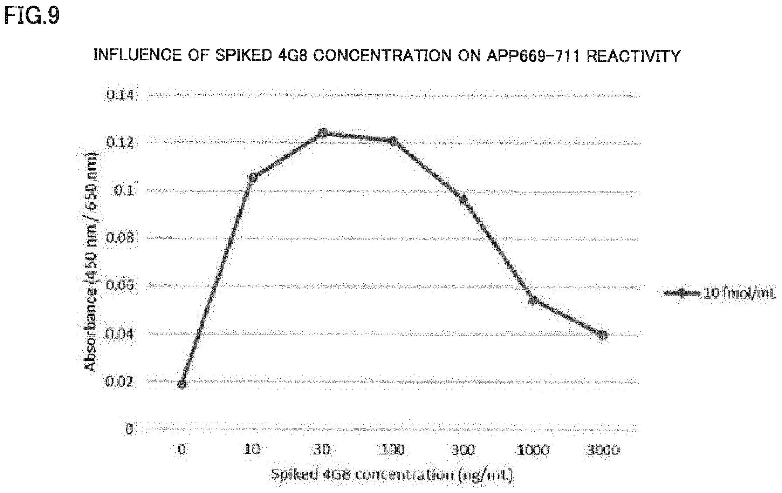

Fig. 9 shows results for APP669-711 in Example 3, a graph showing APP669-711 ELISA reactivity when the concentration of anti-Aβ antibody clone 4G8 in the sample diluent was 0, 10, 30, 100, 300, 1000, or 3000 ng/mL, where the horizontal axis represents the concentration (ng/mL) of anti-Aβ antibody clone 4G8 in the sample diluent, and the vertical axis represents the absorbance at 450 nm/650 nm.

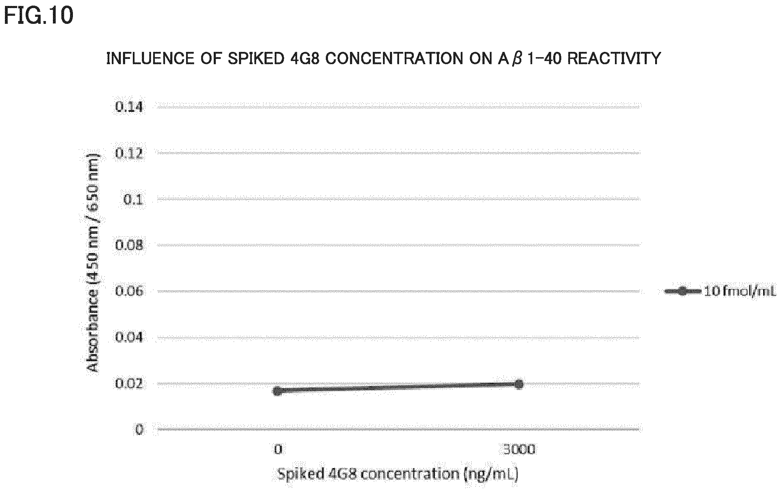

Fig. 10 shows results for Aβ1-40 in Example 3, a graph showing Aβ1-40 ELISA reactivity when the concentration of anti-Aβ antibody clone 4G8 in the sample diluent was 0, 3000 ng/mL, where the horizontal axis represents the concentration (ng/mL) of anti-Aβ antibody clone 4G8 in the sample diluent, and the vertical axis represents the absorbance at 450 nm/650 nm.

### DESCRIPTION OF EMBODIMENTS

### [1. APP669-711-N-Terminus-Recognizing Monoclonal Antibody]

An APP669-711-N-terminus-recognizing monoclonal antibody (a first antibody) used in the present invention recognizes immunospecifically the N terminus of an APP669-711 peptide.

Preparation of the antibody capable of recognizing the N terminus of APP669-711 was conducted by immunizing a mouse with a KLH-bound synthetic peptide VKMC (SEQ ID NO: 5) and then performing cell fusion and screening to obtain a hybridoma that produces an antibody capable of recognizing the N terminus of APP669-711. Details of the antibody preparation will be described in Examples.

This antibody is an antibody capable of recognizing the N terminus of APP669-711, and is therefore capable of specifically recognizing the N termini of various peptides belonging to the group of APP669-x, including APP669-711 (SEQ ID NO: 3). Using this APP669-711-N-terminus-recognizing monoclonal antibody can cause immune reaction between APP669-711 in the sample and this APP669-711-N-terminus-recognizing monoclonal antibody.

Amyloid precursor protein (APP) is a single-pass transmembrane protein made up of 770 amino acid residues. Amyloid precursor protein (APP) undergoes proteolysis by β-secretase and γ-secretase, and this proteolysis produces amyloid beta peptide (Aβ). APP672-711 and Aβ1-40 represent the same peptide (SEQ ID NO: 1). APP672-713 and Aβ1-42 represent the same peptide (SEQ ID NO: 2). APP669-711 (SEQ ID NO: 3) belongs to the group of APP669-x, where the lower limit to x is greater than 669 (greater than 677, for example) and the upper limit is not particularly limited, being equal to or smaller than 770. However, considering the purport of the present invention, more specifically the purport that an APP669-x-N-terminus-recognizing monoclonal antibody is capable of specifically recognizing the N terminus of an APP669-x peptide to cause immune reaction, the upper limit to x is not particularly limited. Examples of the APP669-x peptide include APP669-711 (SEQ ID NO: 3), APP669-709, APP669-710, and APP669-713.
APP672-711 (Aβ1-40) (SEQ ID NO: 1):
   DAEFRHDSGYEVHHQKLVFFAEDVGSNKGAIIGLMVGGVV
APP672-713 (Aβ1-42) (SEQ ID NO: 2):
   DAEFRHDSGYEVHHQKLVFFAEDVGSNKGAIIGLMVGGVVIA
APP669-711 (SEQ ID NO: 3):
   VKMDAEFRHDSGYEVHHQKLVFFAEDVGSNKGAIIGLMVGGVV
KLH-bound synthetic peptide (SEQ ID NO: 5):
   VKMC

### [2. Immunoassay Method]

A first aspect of the present invention relates to a method of measuring APP669-711 in a sample, comprising:
an immobilization step involving immobilizing, to a support, a first antibody capable of immunospecifically binding to an N terminus of APP669-711;
a blocking step involving blocking the support to which the first antibody is immobilized, with a blocking agent;
a first reaction step involving bringing APP669-711 in the sample into contact with the first antibody thus immobilized, and thereby allowing APP669-711 to bind to the first antibody;
a sample removal step involving removing the sample that is not bound;
a second reaction step involving bringing a second antibody capable of immunospecifically binding to a C terminus of APP669-711, into contact with APP669-711 bound to the first antibody thus immobilized, and thereby allowing the second antibody to bind to APP669-711;
a second antibody removal step involving removing the second antibody that is not bound; and
a detection step involving detecting the presence of the second antibody bound to APP669-711.

In the sandwich immunoassay method according to an aspect of the present invention, the APP669-711-N-terminus-recognizing monoclonal antibody (a first antibody), and a monoclonal antibody or a polyclonal antibody capable of recognizing a C terminus of APP669-711 (a second antibody) can be used.

Fig. 3 is a schematic view of a sandwich ELISA method for APP669-711. Referring to Fig. 3, the N terminus of APP669-711 is captured by an antibody fixed on a microplate capable of specifically recognizing the N terminus of APP669-711 (a first antibody), and the C terminus of APP669-711 is bound to a detection antibody capable of specifically recognizing the C terminus of APP669-711 (a second antibody). Detecting this detection antibody enables measuring APP669-711.

The C terminus of APP669-711 is the same as the C terminus of Aβ1-40, and an antibody capable of recognizing the C terminus (the second antibody) is commercially available.

Examples of the sandwich immunoassay method include enzyme immunoassay method (EIA), radioimmunoassay method (RIA), chemiluminescence immunoassay method (CIA), fluorescence immunoassay method (FIA), electrochemiluminescence immunoassay method (ECLIA), bioluminescence immunoassay method (BLIA), immuno-PCR, turbidimetric immunoassay (TAI), and latex agglutination turbidimetry (LA).

In the sandwich immunoassay method, an immobilization step is performed, which involves immobilizing (fixing), to a support, a first antibody capable of immunospecifically binding to an N terminus of APP669-711.

In the immobilization step, in accordance with an ordinary procedure, a first antibody solution containing the first antibody may be brought into contact with a surface of the support, allowing the first antibody to fix to the support.

The concentration of the first antibody in the first antibody solution is not particularly limited, but it may be, for example, from 2.5 to 25 µg/mL, or from 5 to 20 µg/mL. The pH of the first antibody solution may be neutral or weak alkaline (about 7 to 10, for example). Sodium bicarbonate buffer (pH9.6) or phosphate buffer (pH7.4) can be used, for example.

The first antibody solution used for immobilization may be removed from the support, followed by washing as appropriate. The temperature, the time, and the like for immobilization may be determined in accordance with an ordinary procedure.

After the immobilization step, a blocking step is performed, which involves blocking the support to which the first antibody is immobilized, with a blocking agent. Blocking is performed for reducing non-specific binding of a target peptide to the surface of the support.

In the blocking step, a blocking solution containing the blocking agent may be brought into contact with the surface of the support to which the first antibody is immobilized, allowing the blocking agent to be adsorbed to the surface of the support.

In the blocking step, a blocking agent that is generally used can be used. Examples of the blocking agent include bovine serum protein and milk protein. Among these, in the sandwich immunoassay method for APP669-711, milk protein is preferable.

The concentration of the milk protein in the blocking solution is not particularly limited, but it may be, for example, 2 mg/mL or more, or from 2.5 to 10 mg/mL. The concentration may be greater than 10 mg/mL, such as about 15 mg/mL or about 20 mg/mL, for example.

The blocking solution used for blocking may be removed from the support, followed by washing as appropriate. The temperature, the time, and the like for blocking may be determined in accordance with an ordinary procedure.

In the sandwich immunoassay method, before the first reaction step, a milk protein addition step may be performed, which involves adding milk protein to the sample. Adding milk protein to the sample can reduce non-specific binding of a target peptide to the surface of the support, in other words, can assist the blocking step.

In the milk protein addition step, the sample may be diluted with a diluent containing the milk protein in a concentration of 2 mg/mL or more, or from 2 to 4 mg/mL. The concentration may be greater than 4 mg/mL, such as about 10 mg/mL or about 20 mg/mL, for example. Regarding the dilution rate, the dilution rate usable for dilution of a reference material for the purpose of calibration curve generation may allow the relationship between the measurement data such as absorbance and the concentration of the reference material to be expressed in the form of a straight line or a curved line; and the dilution rate usable for dilution of a biological sample may allow the measurement data such as absorbance to fit within the range of the calibration curve.

Further, in the sandwich immunoassay method, before the first reaction step, an antigen affinity substance addition step may be further performed, which involves adding an antigen affinity substance capable of binding to APP669-711 to the sample.

In this case, either of the above steps may be performed first, that is, the step of adding milk protein to the sample and the step of adding the antigen affinity substance to the sample, or these steps may be performed at the same time.

Adding an antigen affinity substance capable of binding to the target polypeptide, APP669-711, to the sample causes a change in the conformation of the target polypeptide, increasing the spatial distance between the two epitopes to which the two antibodies used in the sandwich immunoassay method are to bind respectively. As a result, antigen-antibody reaction at both sites proceeds well, increasing the sensitivity of the detection and quantification of the target polypeptide.

The antigen affinity substance may be a substance having an affinity for target polypeptide APP669-711 and capable of binding thereto, and examples thereof include antibodies, peptides, low-molecular compounds, and nucleic acid aptamers. The "binding" here includes bindings caused by intermolecular interactions such as electrostatic interaction, the van der Waals forces, hydrogen bonding, hydrophobic interaction, dipole interaction, and dispersion force. It may be any binding as long as it can cause a change in the conformation of target polypeptide APP669-711 and thereby increase the spatial distance between the two epitopes to which the two antibodies used in the sandwich immunoassay method are to bind respectively.

An antibody for use as the antigen affinity substance may be an antibody having an antigen-binding site that is different from the antigen-binding sites of the first antibody and the second antibody.

A peptide for use as the antigen affinity substance may be a peptide consisting of 2 to 12 amino acid residues, and examples of a peptide capable of binding to Aβ, for example, include iAβ5 (5 amino acids), D3 (12 amino acids), and NH2-D-Trp-Aib-OH (2 amino acids).

A low-molecular compound for use as the antigen affinity substance may be a low-molecular compound that is capable of binding to the target polypeptide, and examples thereof include low-molecular compounds capable of binding to a certain protein, such as those used in drug discovery. Examples of low-molecular compounds capable of binding to Aβ include Scyllo-inositol. Various inhibitors (for example, various low-molecular compounds under the Stemolecule (trade mark) series) may also be used.

Examples of nucleic acid aptamers for use as the antigen affinity substance include DNA aptamers and RNA aptamers.

The amount of the antigen affinity substance to be added is dependent on the amount of target polypeptide APP669-711 in the sample and the type of the antigen affinity substance, and is not particularly limited as long as the amount allows for binding to the target polypeptide and thereby changes the conformation of the target polypeptide. For example, the amount of the antigen affinity substance to be added may be from about 0.1 ng/mL to 100000 ng/mL, preferably from about 0.1 ng/mL to 10000 ng/mL, more preferably from about 0.1 ng/mL to 3000 ng/mL, in terms of the concentration thereof in the sample containing target polypeptide APP669-711.

In this case, preferably, the antigen affinity substance acts upon neither near the N terminus of target polypeptide APP669-711 nor near the C terminus of the target polypeptide. It is not preferable that the antigen affinity substance compete or interfere with the first antibody or the antigen affinity substance compete or interfere with the second antibody, vying for the target polypeptide.

More specifically, the antigen affinity substance preferably acts upon an intermediate area between the fourth residue from the N terminus of target polypeptide APP669-711 and the fourth residue from the C terminus. Further, the antigen affinity substance preferably acts upon an intermediate area between the sixth residue from the N terminus of target polypeptide APP669-711 and the sixth residue from the C terminus.

When sandwich ELISA (Enzyme-Linked ImmunoSorbent Assay) using an enzyme as a label substance is employed, the antibody capable of recognizing the N terminus of the target polypeptide (the first antibody) may be used as a fixing antibody (a capture antibody), and the antibody capable of recognizing the C terminus of the target polypeptide (the second antibody) may be used as a detection antibody (a labelling antibody).

The sandwich immunoassay method according to the present invention is applicable not only to sandwich ELISA using an enzyme as a label substance, but also to radioimmunoassay method (RIA) using a radioisotope, chemiluminescence immunoassay method (CIA) using a chemiluminescent substance, fluorescence immunoassay method (FIA) using a fluorescent substance, electrochemiluminescence immunoassay method (ECLIA) using a metal complex, bioluminescence immunoassay method (BLIA) using a bioluminescent substance such as luciferase, immuno-PCR involving PCR-amplification of an antibody-labeled nucleic acid for detection, turbidimetric immunoassay method (TAI) involving detection of turbidity caused by immune complex formation, latex agglutination turbidimetry (LA) involving detection of aggregated latex generated by immune complex formation, and immunochromatography method involving reaction on a cellulose membrane.

The basic operation of the sandwich immunoassay method according to the present invention, including when adding the antigen affinity substance to the sample, can be implemented in accordance with a known operation.

In the present invention, target APP669-711 peptide is contained in the biological sample. The biological sample includes body fluids such as blood, cerebrospinal fluid (CSF), urine, biological secretory fluid, saliva, and sputum; and stool. The blood sample includes whole blood, plasma, serum, and the like. The blood sample can be prepared by treating, as appropriate, whole blood collected from an individual. The treatment for preparing the blood sample from collected whole blood is not particularly limited, and it may be any clinically acceptable treatment. For example, centrifugation and/or the like may be adopted. The blood sample may be once preserved as appropriate at a low temperature (such as by freezing) during or after the preparation. In the present invention, the biological sample is discarded, not returned to the individual from which it was collected. Using a blood sample as a target sample is preferable because collecting this type of sample is minimally invasive compared to collecting solid or cerebrospinal fluid, and also because a blood sample is used in ordinary medical examination and in comprehensive medical examination, etc. for screening for various diseases.

### [3. Immunoassay Kit]

A second aspect of the present invention relates to a kit for measuring APP669-711, comprising:
an APP669-711-N-terminus-recognizing monoclonal antibody capable of specifically recognizing an N terminus of APP669-711 (a first antibody);
a monoclonal antibody or a polyclonal antibody capable of recognizing a C terminus of APP669-711 (a second antibody); and
a blocking agent.

This measurement kit may further comprise an antigen affinity substance capable of binding to APP669-711. Preferably, the blocking agent is milk protein.

The kit can also include various components used in the operation of the sandwich immunoassay method, such as, for example, a diluent for sample solution preparation and/or a washing solution.

### [Examples]

In the following, a specific description will be given of the present invention, referring to examples. However, the present invention is not limited to these examples. The amount of a component shown below in % is a weight-based amount when the component is solid, or a volume-based amount when the component is liquid, unless otherwise specified.

### [Experiment Example 1: Preparation of APP669-711-N-Terminus-Recognizing Antibody]

### [Experiment Example 1-1: Preparation of Monoclonal Antibody]

Cys at the C terminus of a synthetic peptide VKMC (SEQ ID NO: 4) was brought to bind to a carrier protein (KLH), with the use of a divalent reactive reagent.

FCA (Freund's Complete Adjuvant) and a syringe were used to mix and emulsify this KLH-bound synthetic peptide, which was then injected (for immunization) into BALB/c mice intramuscularly at the tail base in an amount of 200 µg per mouse. Further, the antigen was mixed and emulsified with the use of FICA (Freund's Incomplete Adjuvant), and subcutaneously injected (for booster immunization) twice, 2 weeks apart, in an amount of 50 µg per mouse. Subsequently, final immunization was performed intraperitoneally in an amount of 100 µg per mouse.

Three days after the final immunization, the spleen was taken out, followed by lymphocyte separation and then cryopreservation at -80°C. Separately, whole blood was drawn from each mouse, followed by serum separation from the blood and then cryopreservation at -40°C.

The lymphocytes and mouse myeloma thus obtained were subjected to cell fusion in the presence of 50% polyethylene glycol. The fused cells were dispensed in eight 96-well microplates and cultured. Eight days after the cell fusion, culture supernatant was sampled from each well of the 96-well micro-well plates, followed by sequential primary and secondary ELISA screenings, and then cloning cells in positive wells by limiting dilution. Subsequently, primary and secondary ELISA screenings were performed sequentially, followed by cryopreservation of hybridomas of positive clones.

Each hybridoma (clone 20-1A, 24-6G, or 34-6E) thus obtained was subjected to high-density serum-free culture. From the culture supernatant, an antibody was purified with the use of Protein A.

### [Experiment Example 1-2: Checking Specificity of Monoclonal Antibody by Direct ELISA]

Specificity of APP669-711-N-terminus-recognizing antibody clones 20-1A, 24-6G, 34-6E was evaluated by direct ELISA. Direct ELISA was performed as below.

Synthetic peptides APP669-711 (Peptide Institute), Aβ1-40 (Peptide Institute), and APP668-677 (SEQ ID NO: 4) (Toray Research Center) were diluted with sodium bicarbonate buffer (pH9.6) to concentrations of 50 and 500 pmol/mL. The sequences of the synthetic peptides are shown in Table 1. Each synthetic peptide solution was added in an amount of 50 µL to each well of a 96-well microplate, and incubated at 4°C for 2 hours for fixation. A blank well was also prepared. The solution in the plate was removed, and a 4-fold diluted Block Ace (DS Pharma) was added in an amount of 100 µL per well, followed by incubation at 4°C for 2 hours for blocking. The solution in the plate was removed, followed by washing with 300 µL of PBST (0.05% Tween 20 in PBS). An APP669-x-N-terminus-recognizing antibody in a concentration of 0.5 µg/mL, obtained by dilution with a 10-fold diluted Block Ace, was added in an amount of 50 µL per well, followed by incubation at 4°C for 1 hour. The sample solution in the plate was removed, followed by washing with 300 µL of PBST. A solution of HRP-conjugated anti-mouse IgG antibody (ZYMED) that was 4000-fold diluted with a 10-fold diluted Block Ace was added in an amount of 50 µL per well, followed by incubation at 4°C for 1 hour. The solution in the plate was removed, followed by washing with 300 µL of PBST. ELISA POD Substrate TMB Kit (Nacalai) was added in an amount of 100 µL, followed by incubation in dark for 30 minutes for color development. The color development reaction was terminated by addition of 100 µL of 2 N sulfuric acid. The absorbance at 450 nm (primary wavelength)/650 nm (secondary wavelength) was measured with a microplate reader.

These results are shown in Figs. 4 to 6. More specifically, Fig. 4 is a graph showing results of direct ELISA using APP669-711-N-terminus-specific antibody clone 34-6E. Fig. 5 is a graph showing results of direct ELISA using APP669-711-N-terminus-specific antibody clone 24-6G. Fig. 6 is a graph showing results of direct ELISA using APP669-711-N-terminus-specific antibody clone 20-1A. In Figs. 4 to 6, the horizontal axis represents the peptide concentration (pmol/mL), and the vertical axis represents the absorbance at 450 nm (primary wavelength)/650 nm (secondary wavelength) measured with a microplate reader.

Referring to Figs. 4 to 6, it was demonstrated that all APP669-711-N-terminus-recognizing antibody clones 20-1A, 24-6G, 34-6E successfully reacted with APP669-711. In contrast, no reactivity was found for Aβ1-40 or APP668-677. APP669-711 has the same amino acid sequence as Aβ1-40 except it has three amino acids longer than Aβ1-40 at the N terminus, but it did not react with Aβ1-40, indicating that clones 20-1A, 24-6G, 34-6E recognize these three amino acids at the N terminus of APP669-711. Further, APP668-677 is only a single amino acid longer than APP669-711 at the N terminus, but it did not react with clones 20-1A, 24-6G, 34-6E. This shows that clones 20-1A, 24-6G, 34-6E specifically recognize the N terminus of APP669-711.

The above results demonstrated that the three APP669-711-N-terminus-recognizing antibody clones specifically recognize the N terminus of APP669-711.

**[Table 1]**

| SEQ ID NO: | Peptide name | Sequence |
|---|---|---|
| 1 | Aβ1-40 | DAEFRHDSGYEVHHQKLVFFAEDVGSNKGAIIGLMVGGVV |
| 2 | Aβ1-42 | DAEFRHDSGYEVHHQKLVFFAEDVGSNKGAIIGLMVGGVVIA |
| 3 | APP669-711 | VKMDAEFRHDSGYEVHHQKLVFFAEDVGSNKGAIIGLMVGGVV |
| 4 | APP668-677 | EVKMDAEFRH |

### [Example 1: Study of Concentration of Capture Antibody]

To determine a suitable concentration of a capture antibody (a first antibody), fixation (immobilization) to the surface of a support was performed with varied concentrations of a capture antibody solution, and ELISA reactivity was evaluated.

An APP669-711-N-terminus-recognizing antibody (clone 34-6E) as a capture antibody was diluted with sodium bicarbonate buffer (pH9.6) to a concentration of 2.5, 5, 10, or 20 µg/mL, and the antibody solution was added to each well of a 96-well plate in an amount of 50 µL per well, followed by incubation at 4°C for 6 hours to fix the capture antibody. The antibody solution in the plate was removed, and 20% Blocking One (Nacalai Tesque) was added in an amount of 100 µL per well, followed by incubation at 4°C for 2 hours for blocking. APP669-711 standard liquid was diluted with a reaction liquid (5% Blocking One in PBST, containing 200 ng/mL of anti-Aβ antibody clone 4G8) to prepare APP669-711 standard liquids of various concentrations. The 20% Blocking One in the plate was removed, followed by washing three times with 300 µL of PBST. The APP669-711 standard liquid was added to the plate in an amount of 50 µL per well, followed by incubation at 4°C overnight. The APP669-711 standard liquid in the plate was removed, followed by washing three times with 300 µL of PBST.

A solution of HRP-conjugated antibody capable of recognizing the C terminus (clone BA27) contained in a Human βAmyloid (1-40) ELISA Kit (Wako Pure Chemical Industries) was added in an amount of 50 µL per well, followed by incubation at 4°C for 2 hours. The solution in the plate was removed, followed by washing 5 times with 300 µL of PBST. ELISA POD Substrate TMB Kit (Nacalai) was added in an amount of 100 µL, followed by incubation in dark for 15 minutes for color development. The color development reaction was terminated by addition of 100 µL of 2 N sulfuric acid. A microplate reader was used to measure the absorbance at 450 nm (primary wavelength)/650 nm (secondary wavelength).

Results are shown in Fig. 7. Fig. 7 is a graph showing APP669-711 ELISA reactivity where the concentration of a capture antibody solution during a step of fixing an APP669-711-N-terminus-recognizing antibody was changed, where the horizontal axis represents the concentration (fmol/mL) of APP669-711 standard liquid, and the vertical axis represents the absorbance at 450 nm/650 nm.

When the concentration of the N-terminus-recognizing antibody (clone 34-6E) was increased from 2.5 µg/mL to 5 µg/mL, the absorbance for an APP669-711 concentration of 80 fmol/mL increased by 43% (Fig. 7). Further, when it was increased from 5 µg/mL to 10 µg/mL, the increase was small, by 9%. This indicates that a preferable concentration of the N-terminus-recognizing antibody is 5 µg/mL or more.

### [Example 2: Study of pH During Fixation of Capture Antibody]

Fixation (immobilization) of a capture antibody (a first antibody) to the surface of a support was performed when the pH of a capture antibody solution was 9.6 or 7.4, and ELISA reactivity was evaluated.

An APP669-711-N-terminus-recognizing antibody (clone 34-6E) as a capture antibody was diluted with sodium bicarbonate buffer (pH9.6) or phosphate buffer (pH7.4) to a concentration of 20 µg/mL, and the antibody solution was added to each well of a 96-well plate in an amount of 50 µL per well, followed by incubation at 4°C for 6 hours to fix the capture antibody. The antibody solution in the plate was removed, and 20% Blocking One (Nacalai Tesque) was added in an amount of 100 µL per well, followed by incubation at 4°C for 2 hours for blocking. APP669-711 standard liquid or Aβ1-40 standard liquid was diluted with a sample diluent (5% Blocking One in PBST, containing 200 ng/mL of anti-Aβ antibody clone 4G8) to prepare APP669-711 standard liquids of various concentrations. The 20% Blocking One in the plate was removed, followed by washing three times with 300 µL of PBST. The APP669-711 standard liquid was added to the plate in an amount of 50 µL per well, followed by incubation at 4°C overnight. The APP669-711 standard liquid in the plate was removed, followed by washing three times with 300 µL of PBST.

A solution of HRP-conjugated antibody capable of recognizing the C terminus (clone BA27) was added in an amount of 50 µL per well, followed by incubation at 4°C for 2 hours. The solution in the plate was removed, followed by washing 5 times with 300 µL of PBST. ELISA POD Substrate TMB Kit (Nacalai) was added in an amount of 100 µL, followed by incubation in dark for 15 minutes for color development. The color development reaction was terminated by addition of 100 µL of 2 N sulfuric acid. A microplate reader was used to measure the absorbance at 450 nm (primary wavelength)/650 nm (secondary wavelength).

Results are shown in Fig. 8. Fig. 8 is a graph showing APP669-711 ELISA reactivity when the pH of a capture antibody solution was 9.6 or 7.4 during a step of fixing an APP669-711-N-terminus-recognizing antibody, where the horizontal axis represents the concentration (fmol/mL) of APP669-711 standard liquid, and the vertical axis represents the absorbance at 450 nm/650 nm.

Changing the pH of the antibody solution during fixation of the N-terminus-recognizing antibody between 9.6 and 7.4 did not affect the absorbance. In other words, it was demonstrated that the fixation of the N-terminus-recognizing antibody can occur whether the antibody solution is neutral or weak alkaline (Fig. 8).

### [Example 3: Effect of Addition of Anti-Aβ Antibody to Sample Diluent]

To a diluent for diluting a sample containing antigen APP669-711, an anti-Aβ antibody as an antigen affinity substance was added, and ELISA reactivity was evaluated.

An APP669-711-N-terminus-recognizing antibody (clone 34-6E) as a capture antibody was diluted with sodium bicarbonate buffer (pH9.6) to a concentration of 20 µg/mL, and the antibody solution was added to each well of a 96-well plate in an amount of 50 µL per well, followed by incubation at 4°C for 6 hours to fix the capture antibody. The antibody solution in the plate was removed, and 20% Blocking One (Nacalai Tesque) was added in an amount of 100 µL per well, followed by incubation at 4°C for 2 hours for blocking. APP669-711 standard liquid or Aβ1-40 standard liquid was diluted with a sample diluent (5% Blocking One in PBST, containing anti-Aβ antibody clone 4G8 in various concentrations) to prepare APP669-711 standard liquids (APP669-711 concentration, 10 fmol/mL) or Aβ1-40 standard liquids (Aβ1-40 concentration, 10 fmol/mL) containing the anti-Aβ antibody in various concentrations. The 20% Blocking One in the plate was removed, followed by washing three times with 300 µL of PBST. The APP669-711 standard liquid or the Aβ1-40 standard liquid was added to the plate in an amount of 50 µL per well, followed by incubation at 4°C overnight. The APP669-711 standard liquid or the Aβ1-40 standard liquid in the plate was removed, followed by washing three times with 300 µL of PBST.

A solution of HRP-conjugated antibody capable of recognizing the C terminus (clone BA27) was added in an amount of 50 µL per well, followed by incubation at 4°C for 8 hours. The solution in the plate was removed, followed by washing 5 times with 300 µL of PBST. ELISA POD Substrate TMB Kit (Nacalai) was added in an amount of 100 µL, followed by incubation in dark for 15 minutes for color development. The color development reaction was terminated by addition of 100 µL of 2 N sulfuric acid. A microplate reader was used to measure the absorbance at 450 nm (primary wavelength)/650 nm (secondary wavelength).

Results are shown in Fig. 9 and Fig. 10. Fig. 9 shows results for APP669-711, a graph showing APP669-711 ELISA reactivity when the concentration of anti-Aβ antibody clone 4G8 in the sample diluent was 0, 10, 30, 100, 300, 1000, or 3000 ng/mL, where the horizontal axis represents the concentration (ng/mL) of anti-Aβ antibody clone 4G8 in the sample diluent, and the vertical axis represents the absorbance at 450 nm/650 nm. Fig. 10 shows results for Aβ1-40, a graph showing Aβ1-40 ELISA reactivity when the concentration of anti-Aβ antibody clone 4G8 in the sample diluent was 0, 3000 ng/mL, where the horizontal axis represents the concentration (ng/mL) of anti-Aβ antibody clone 4G8 in the sample diluent, and the vertical axis represents the absorbance at 450 nm/650 nm.

The absorbance increased when anti-Aβ antibody 4G8 was added to the sample diluent, as compared to when anti-Aβ antibody 4G8 was not added (namely, 0 ng/mL) (Fig. 9). As the cross-reactivity with Aβ1-40 was checked when anti-Aβ antibody 4G8 was added to the sample diluent, and no cross-reaction was observed, proving the specificity to APP669-711 (Fig. 10). As shown by the above results, addition of anti-Aβ antibody 4G8 to the sample diluent was useful for enhancing APP669-711 ELISA reactivity.

### [Example 4: Study of Blocking Agent]

Two reagents, namely, Blocking One (Nacalai Tesque) containing bovine serum protein and Block Ace (Snow Brand) containing milk protein, were used as a blocking agent, and the influence of an interfering substance in a plasma sample on measurement results was examined by a spike and recovery test.

An APP669-711-N-terminus-recognizing antibody (clone 34-6E) as a capture antibody was diluted with sodium bicarbonate buffer (pH9.6) to a concentration of 20 µg/mL, and the antibody solution was added to each well of a 96-well plate in an amount of 50 µL per well, followed by incubation at 4°C for 6 hours to fix the capture antibody. The antibody solution in the plate was removed, and 20% Blocking One or 10 mg/mL Block Ace was added in an amount of 100 µL per well, followed by incubation at 4°C for 2 hours for blocking. APP669-711 standard liquid was diluted with either of two sample diluents (5% Blocking One in PBST or 4 mg/mL Block Ace in PBST, containing 120 ng/mL anti-Aβ antibody clone 4G8) to prepare APP669-711 standard liquid for calibration curve creation. Spiked samples were prepared, by diluting human plasma 4-fold with either of two sample diluents or by spiking APP669-711 of various concentrations to the plasma samples. The blocking agent in the plate was removed, followed by washing three times with 300 µL of PBST. The APP669-711 standard liquid and the human plasma sample were added to the plate in an amount of 50 µL per well, followed by incubation at 4°C overnight. The APP669-711 standard liquid and the human plasma sample in the plate were removed, followed by washing three times with 300 µL of PBST.

A solution of HRP-conjugated antibody capable of recognizing the C terminus (clone BA27) was added in an amount of 50 µL per well, followed by incubation at 4°C for 2 hours. The solution in the plate was removed, followed by washing 5 times with 300 µL of PBST. ELISA POD Substrate TMB Kit (Nacalai) was added in an amount of 100 µL, followed by incubation in dark for 15 minutes for color development. The color development reaction was terminated by addition of 100 µL of 2 N sulfuric acid. A microplate reader was used to measure the absorbance at 450 nm (primary wavelength)/650 nm (secondary wavelength).

A ratio of an actual value to a theoretical value was determined for each known concentration of APP669-711 spiked to plasma, to determine a spike and recovery rate (Table 2). As a result, the average recovery rate for Blocking One was 67%, and that for Block Ace was 90%. This shows that, compared to Blocking One containing bovine serum protein, Block Ace containing milk protein can reduce the influence of an interfering substance in plasma on the quantified value.

**[Table 2]**

| Blocking agent | Spiked APP669-711 Conc. (fmol/mL) | Actual value (fmol/mL) | Theoretical value (fmol/mL) | Spike and recovery rate | Average recovery rate |
|---|---|---|---|---|---|
| Blocking One | 0 | 1.00 | | | |
| | 5 | 4.09 | 6.00 | 68% | |
| | 10 | 7.20 | 11.00 | 66% | |
| | 20 | 14.29 | 21.00 | 68% | 67% |
| Block Ace | 0 | 3.68 | | | |
| | 5 | 7.73 | 8.68 | 89% | |
| | 10 | 12.47 | 13.68 | 91% | |
| | 20 | 21.09 | 23.68 | 89% | 90% |

### [Example 5: Study of Blocking Agent Concentration in Blocking Step]

To determine a suitable concentration of Block Ace (Snow Brand) in the blocking solution in the blocking step, a spike and recovery test was performed with varied concentrations of the blocking solution.

An APP669-711-N-terminus-recognizing antibody (clone 34-6E) as a capture antibody was diluted with sodium bicarbonate buffer (pH9.6) to a concentration of 20 µg/mL, and the antibody solution was added to each well of a 96-well plate in an amount of 50 µL per well, followed by incubation at 4°C for 6 hours to fix the capture antibody. The antibody solution in the plate was removed, and 2.5, 5, or 10 mg/mL Block Ace was added in an amount of 100 µL per well, followed by incubation at 4°C for 2 hours for blocking. APP669-711 standard liquid was diluted with a sample diluent (4 mg/mL Block Ace in PBST, containing 120 ng/mL of anti-Aβ antibody clone 4G8) to prepare APP669-711 standard liquid for calibration curve creation. Spiked samples were prepared, by diluting human plasma 4-fold with the sample diluent or by spiking APP669-711 of various concentrations to the plasma sample. The blocking agent in the plate was removed, followed by washing three times with 300 µL of PBST. The APP669-711 standard liquid and the human plasma sample were added to the plate in an amount of 50 µL per well, followed by incubation at 4°C overnight. The APP669-711 standard liquid and the human plasma sample in the plate were removed, followed by washing three times with 300 µL of PBST.

A solution of HRP-conjugated antibody capable of recognizing the C terminus (clone BA27) was added in an amount of 50 µL per well, followed by incubation at 4°C for 2 hours. The solution in the plate was removed, followed by washing 5 times with 300 µL of PBST. ELISA POD Substrate TMB Kit (Nacalai) was added in an amount of 100 µL, followed by incubation in dark for 15 minutes for color development. The color development reaction was terminated by addition of 100 µL of 2 N sulfuric acid. A microplate reader was used to measure the absorbance at 450 nm (primary wavelength)/650 nm (secondary wavelength).

A ratio of an actual value to a theoretical value was determined for each known concentration of APP669-711 added to plasma, to determine a spike and recovery rate (Table 3). As a result, the average recovery rates for all the Block Ace concentrations in the blocking solution were 90% or more, indicating successful quantification.

**[Table 3]**

| Block Ace concentration | Spiked APP669-711 Conc. (fmol/mL) | Actual value (fmol/mL) | Theoretical value (fmol/mL) | Spike and recovery rate | Average recovery rate |
|---|---|---|---|---|---|
| 10 mg/mL | 0 | 3.05 | | | |
| | 5 | 7.11 | 8.05 | 88% | |
| | 10 | 12.43 | 13.05 | 95% | |
| | 20 | 21.44 | 23.05 | 93% | 92% |
| 5 mg/mL | 0 | 3.22 | | | |
| | 5 | 7.71 | 8.22 | 94% | |
| | 10 | 12.49 | 13.22 | 94% | |
| | 20 | 21.53 | 23.22 | 93% | 94% |
| 2.5 mg/mL | 0 | 3.18 | | | |
| | 5 | 7.87 | 8.18 | 96% | |
| | 10 | 12.98 | 13.18 | 98% | |
| | 20 | 21.18 | 23.18 | 91% | 95% |

### [Example 6: Study of Blocking Agent Concentration of Sample Diluent]

To determine a suitable concentration of Block Ace (Snow Brand) in the sample diluent, a spike and recovery test was performed with varied concentrations of Block Ace in the sample diluent.

An APP669-711-N-terminus-recognizing antibody (clone 34-6E) as a capture antibody was diluted with sodium bicarbonate buffer (pH9.6) to a concentration of 20 µg/mL, and the antibody solution was added to each well of a 96-well plate in an amount of 50 µL per well, followed by incubation at 4°C for 6 hours to fix the capture antibody.

The antibody solution in the plate was removed, and 10 mg/mL Block Ace was added in an amount of 100 µL per well, followed by incubation at 4°C for 2 hours for blocking. APP669-711 standard liquid was diluted with a sample diluent (1, 2, or 4 mg/mL Block Ace in PBST, containing 120 ng/mL of anti-Aβ antibody clone 4G8) to prepare APP669-711 standard liquid for calibration curve creation. Spiked samples were prepared, by diluting human plasma 4-fold with the sample diluent or by spiking APP669-711 of various concentrations to the plasma sample. The blocking agent in the plate was removed, followed by washing three times with 300 µL of PBST. The APP669-711 standard liquid and the human plasma sample were added to the plate in an amount of 50 µL per well, followed by incubation at 4°C overnight. The APP669-711 standard liquid and the human plasma sample in the plate were removed, followed by washing three times with 300 µL of PBST.

A solution of HRP-conjugated antibody capable of recognizing the C terminus (clone BA27) was added in an amount of 50 µL per well, followed by incubation at 4°C for 2 hours. The solution in the plate was removed, followed by washing 5 times with 300 µL of PBST. ELISA POD Substrate TMB Kit (Nacalai) was added in an amount of 100 µL, followed by incubation in dark for 15 minutes for color development. The color development reaction was terminated by addition of 100 µL of 2 N sulfuric acid. A microplate reader was used to measure the absorbance at 450 nm (primary wavelength)/650 nm (secondary wavelength).

A ratio of an actual value to a theoretical value was determined for each known concentration of APP669-711 added to plasma, to determine a spike and recovery rate (Table 4). As a result, the lower the concentration of Block Ace in the sample diluent was, the lower the average recovery rate tended to be.

**[Table 4]**

| Block Ace concentration | Spiked APP669-711 Conc. (fmol/mL) | Actual value (fmol/mL) | Theoretical value (fmol/mL) | Spike and recovery rate | Average recovery rate |
|---|---|---|---|---|---|
| 4 mg/mL | 0 | 3.51 | | | |
| | 5 | 7.27 | 8.51 | 85% | |
| | 10 | 11.91 | 13.51 | 88% | |
| | 20 | 20.54 | 23.51 | 87% | 87% |
| 2 mg/mL | 0 | 2.30 | | | |
| | 5 | 6.10 | 7.30 | 84% | |
| | 10 | 10.56 | 12.30 | 86% | |
| | 20 | 18.66 | 22.30 | 84% | 84% |
| 1 mg/mL | 0 | 2.21 | | | |
| | 5 | 5.60 | 7.21 | 78% | |
| | 10 | 9.65 | 12.21 | 79% | |
| | 20 | 17.22 | 22.21 | 78% | 78% |

(1) A method of measuring APP669-711 in a sample, comprising:
   an immobilization step involving immobilizing, to a support, a first antibody capable of immunospecifically binding to an N terminus of APP669-711;
   a blocking step involving blocking the support to which the first antibody is immobilized, with a blocking agent;
   a first reaction step involving bringing APP669-711 in the sample into contact with the first antibody thus immobilized, and thereby allowing APP669-711 to bind to the first antibody;
   a sample removal step involving removing the sample that is not bound;
   a second reaction step involving bringing a second antibody capable of immunospecifically binding to a C terminus of APP669-711, into contact with APP669-711 bound to the first antibody thus immobilized, and thereby allowing the second antibody to bind to APP669-711;
   a second antibody removal step involving removing the second antibody that is not bound; and
   a detection step involving detecting the presence of the second antibody bound to APP669-711.
(2) The method according to (1) above, wherein, in the blocking step, the blocking agent is milk protein.
(3) The method according to (1) or (2) above, wherein, in the blocking step, a blocking solution containing the blocking agent is brought into contact with the support to which the first antibody is immobilized.
(4) The method according to (3) above, wherein, in the blocking step, the blocking agent is milk protein, and a concentration of the milk protein in the blocking solution is from 2.5 to 10 mg/mL.
(5) The method according to any one of (1) to (4) above, further comprising, before the first reaction step, a milk protein addition step involving adding milk protein to the sample.
(6) The method according to (5) above, wherein, in the milk protein addition step, the sample is diluted with a diluent containing the milk protein in a concentration from 2 to 4 mg/mL.
(7) The method according to any one of (1) to (6) above, wherein, in the immobilization step, a first antibody solution containing the first antibody is brought into contact with the support.
(8) The method according to (7) above, wherein a concentration of the first antibody in the first antibody solution is from 5 to 20 µg/mL.
(9) The method according to (7) or (8) above, wherein a pH of the first antibody solution is neutral or weak alkaline.
(10) The method according to any one of (1) to (9) above, further comprising, before the first reaction step, an antigen affinity substance addition step involving adding an antigen affinity substance capable of binding to APP669-711 to the sample.
(11) The method according to any one of (1) to (10) above, wherein the sample is a biologically-derived sample selected from the group consisting of blood, cerebrospinal fluid, urine, stool, and biological secretory fluid.
(12) A kit for measuring APP669-711, comprising:
   an APP669-711-N-terminus-recognizing monoclonal antibody capable of specifically recognizing an N terminus of APP669-711 (a first antibody);
   a monoclonal antibody or a polyclonal antibody capable of recognizing a C terminus of APP669-711 (a second antibody); and
   a blocking agent.
(13) The kit according to (12) above, further comprising an antigen affinity substance capable of binding to APP669-711.
(14) The kit according to (12) or (13) above, wherein the blocking agent is milk protein.

## Claims

1. A method of measuring APP669-711 in a sample, comprising:
an immobilization step involving immobilizing, to a support, a first antibody capable of immunospecifically binding to an N terminus of APP669-711;
a blocking step involving blocking the support to which the first antibody is immobilized, with a blocking agent;
a first reaction step involving bringing APP669-711 in the sample into contact with the first antibody thus immobilized, and thereby allowing APP669-711 to bind to the first antibody;
a sample removal step involving removing the sample that is not bound;
a second reaction step involving bringing a second antibody capable of immunospecifically binding to a C terminus of APP669-711, into contact with APP669-711 bound to the first antibody thus immobilized, and thereby allowing the second antibody to bind to APP669-711;
a second antibody removal step involving removing the second antibody that is not bound; and
a detection step involving detecting the presence of the second antibody bound to APP669-711.

2. The method according to claim 1, wherein, in the blocking step, the blocking agent is milk protein.

3. The method according to claim 1 or 2, wherein, in the blocking step, a blocking solution containing the blocking agent is brought into contact with the support to which the first antibody is immobilized.

4. The method according to claim 3, wherein, in the blocking step, the blocking agent is milk protein, and a concentration of the milk protein in the blocking solution is from 2.5 to 10 mg/mL.

5. The method according to any one of claims 1 to 4, further comprising, before the first reaction step, a milk protein addition step involving adding milk protein to the sample.

6. The method according to claim 5, wherein, in the milk protein addition step, the sample is diluted with a diluent containing the milk protein in a concentration from 2 to 4 mg/mL.

7. The method according to any one of claims 1 to 6, wherein, in the immobilization step, a first antibody solution containing the first antibody is brought into contact with the support.

8. The method according to claim 7, wherein a concentration of the first antibody in the first antibody solution is from 5 to 20 µg/mL.

9. The method according to claim 7 or 8, wherein a pH of the first antibody solution is neutral or weak alkaline.

10. The method according to any one of claims 1 to 9, further comprising, before the first reaction step, an antigen affinity substance addition step involving adding an antigen affinity substance capable of binding to APP669-711 to the sample.

11. The method according to any one of claims 1 to 10, wherein the sample is a biologically-derived sample selected from the group consisting of blood, cerebrospinal fluid, urine, stool, and biological secretory fluid.

12. A kit for measuring APP669-711, comprising:
an APP669-711-N-terminus-recognizing monoclonal antibody capable of specifically recognizing an N terminus of APP669-711 (a first antibody);
a monoclonal antibody or a polyclonal antibody capable of recognizing a C terminus of APP669-711 (a second antibody); and
a blocking agent.

13. The kit according to claim 12, further comprising an antigen affinity substance capable of binding to APP669-711.

14. The kit according to claim 12 or 13, wherein the blocking agent is milk protein.
